(19) Europäisches Patentamt
European Patent Office
Office européen des brevets

(11) Publication number: 0 357 118 B1

(12) **EUROPEAN PATENT SPECIFICATION**

(45) Date of publication of patent specification: **16.06.93**

(51) Int. Cl.5: **C07F 7/10**, C07D 205/085

(21) Application number: **89202044.7**

(22) Date of filing: **03.08.89**

# (54) Beta-lactams synthesis using a metal compound as catalyst.

(30) Priority: **04.08.88 EP 88201692**

(43) Date of publication of application:
**07.03.90 Bulletin 90/10**

(45) Publication of the grant of the patent:
**16.06.93 Bulletin 93/24**

(84) Designated Contracting States:
**AT BE CH DE ES FR GB GR IT LI LU NL SE**

(56) References cited:
**EP-A- 0 180 398**
**EP-A- 0 281 177**

**CHEMICAL ABSTRACTS, vol. 105, no. 25, 22nd December 1986, page 746, abstract no. 226120n, Columbus, Ohio, US; C. WANG et al.: "A stereospecific synthesis of 1-benzyl-3-amido-4-substituted phenyl-2-azetidinones", & HUAXUE XUEBAO 1986, 44(3), 250-4**

**TETRAHEDRON LETTERS, vol. 27, no. 15, 1986, pages 1695-1698, Pergamon Press Ltd, GB; P. ANDREOLI et al.: "A synthetic approach to azetidinones from nitriles and lithiumtriethoxyaluminium hydride"**

**TETRAHEDRON LETTERS, vol. 22, no. 19, 1981, pages 1787-1790, Pergamon Press Ltd, GB; S. DJURIC et al.: "Silicon in synthesis: stabase adducts - A new primary amine protecting group: alkylation of ethyl glycinate"**

(73) Proprietor: **RIJKSUNIVERSITEIT UTRECHT**
**Padualaan 8**
**NL-3584 CH Utrecht(NL)**

(72) Inventor: **Van Koten, Gerard**
**Paltzerweg 180**
**NL-3734 CN Den Dolder(NL)**
Inventor: **Van der Steen, Frederik Hendrik**
**Huetlaan 11**
**NL-3553 GN Utrecht(NL)**
Inventor: **Jastrzebski, Johan Töns Barthold Heinrich**
**Stratusplein 11**
**NL-3731 XA De Bilt(NL)**

(74) Representative: **Huygens, Arthur Victor, Dr. et al**
**c/o Gist-Brocades N.V. Patents & Trademarks Department Wateringseweg 1 PO Box 1**
**NL-2600 MA Delft (NL)**

EP 0 357 118 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to a process for the preparation of trans-β-lactams by reacting an N,N-diprotected glycine ester with an imine in the presence of a metal compound.

Considerable research effort has been, and continues to be expended on the development of new synthetic methods for the production of both new and known antibiotics. The azetidinones or β-lactams have received the most attention in recent years since they are generally well tolerated by humans. The determination of their mode of action on a molecular scale, i.e. their ability to inhibit the synthesis of bacterial cell walls, has further served to stimulate and focus the interest of investigators searching for new synthetic schemes.

In the European patent application EP-A-0281177 a route was described to synthesize 1,3,4-trisubstituted trans-2-azetidinones in which the atom linked to the C-3 carbon atom is nitrogen, as follows: a process was provided for the preparation of trans-β-lactams of formula I'

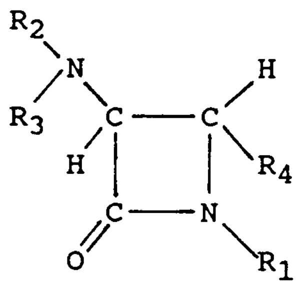

I'

wherein

$R_2$ and $R_3$ are each hydrogen, alkyl, aryl, aralkyl, each optionally substituted with alkyl, aryl or aralkyl, or are

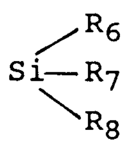

where $R_6$, $R_7$ and $R_8$ are each alkyl, aryl, aralkyl, each optionally substituted and $R_6$, $R_7$ and $R_8$ are the same or different,

and $R_2$ and $R_3$ are the same or different and $R_2$ and $R_3$ are not both methyl or benzyl,

or $R_2$ and $R_3$ together with the nitrogen atom to which they are attached form a ring with up to 8 ring atoms, each optionally substituted with alkyl, aryl or aralkyl,

$R_1$ is hydrogen, halogen, alkyl, alkenyl, alkoxy, aryl or aralkyl, or is

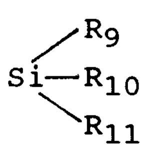

where $R_9$, $R_{10}$ and $R_{11}$ are each alkyl, aryl, aralkyl, each optionally substituted, and $R_9$, $R_{10}$ and $R_{11}$ are the same or different,

$R_4$ is alkyl, hydroxy, halogen, sulfonyl, alkoxy, alkenyl, alkynyl or aryl, each optionally substituted,

or is

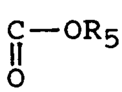

where $R_5$ is alkyl,
by a condensation reaction of a metal enolate

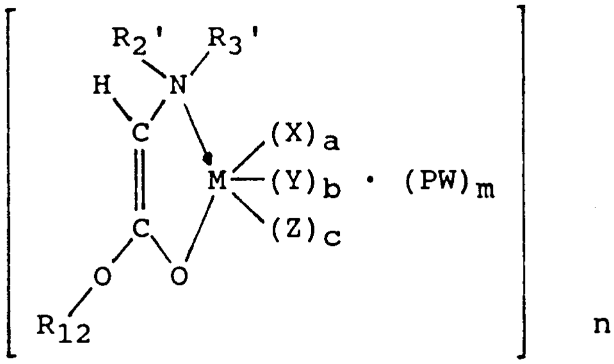

wherein
$R_{12}$ is alkyl, aryl or aralkyl, each optionally substituted with alkyl, aryl or aralkyl,
M is zinc, aluminum, zirconium, boron, tin or titanium,
P is alkali metal,
W, X, Y and Z are alkyl, aryl, halide, alkoxide, thiolate, triflate or any other substituted sulfonate or any other suitable anionic group, and
W, X, Y and Z are the same or different,
and a, b, c and m = 0-1, n = 1-6, all being integers,
$R_2$' and $R_3$' is $R_2$ and $R_3$ respectively, with the proviso that when $R_2$ and $R_3$ are hydrogen, $R_2$' and $R_3$' form together with the nitrogen atom to which they are attached a ring of formula III'

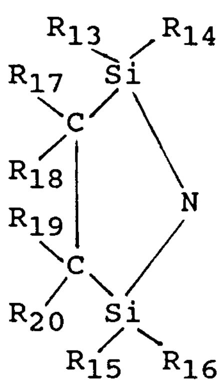

III'

wherein
$R_{13}$, $R_{14}$, $R_{15}$, $R_{16}$, $R_{17}$, $R_{18}$, $R_{19}$ and $R_{20}$ are each alkyl, aryl, aralkyl and $R_{13}$, $R_{14}$, $R_{15}$, $R_{16}$, $R_{17}$, $R_{18}$, $R_{19}$ and $R_{20}$ are the same or different,
with an imine of formula IV'

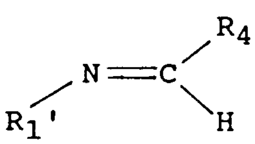

IV'

where $R_4$ is as defined above,
and $R_1$' is $R_1$ with the proviso that when $R_1$ is hydrogen, $R_1$' is

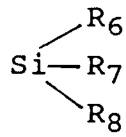

where $R_6$, $R_7$ and $R_8$ are defined as above which group is hydrolysed after the reaction by acid or base, and with the proviso that when $R_2$ and $R_3$ are both hydrogen, the ring of formula III' is converted into $NH_2$ by acid or base catalysed hydrolysis, after the reaction.

To carry out this process as a one-pot process starting from an $R_2$'$R_3$'N-glycine ester (V'), an imine (IV'), an alkali metal base (VI') and a metal compound (VII') was also disclosed in EP-A-0281177. Both the reaction starting from the metal enolate (II') and the one starting from the $R_2$'$R_3$'N-glycine ester (V') and the metal compound (VII') have been displayed in the following scheme (with all variable substituents as defined above):

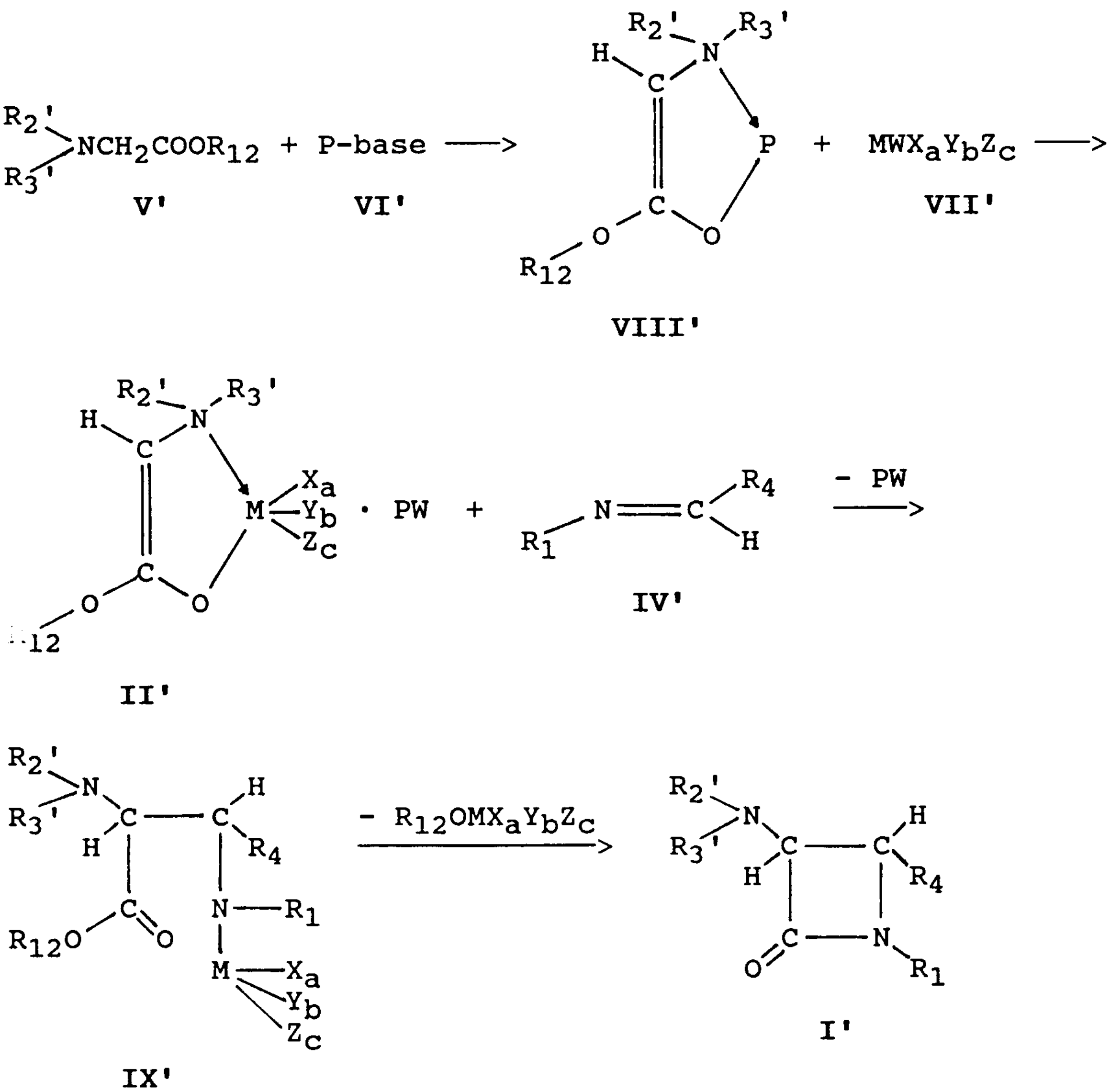


V' VI' VIII' VII' II' IV' IX' I'

The supposed intermediates VIII' and IX' have not been isolated.

Summarizing: besides this above-mentioned one-pot process, EP-A-0281177 discloses the two-step process comprising the isolation of the intermediate zinc enolate (II') and the reaction of this intermediate with an imine (IV') which results in the formation of trans-$\beta$-lactams compounds of formula I'. Therefore equimolar amounts of the metal compound $MW(X)_a(Y)_b(Z)_c$ compared to the other starting materials were

used. Also in the non-prepublished European patent application No. 89201666.8 wherein similar reactions yielding enantioselective compounds have been disclosed, equimolar amounts of the metal compound $MW(X)_a(Y)_b(Z)_c$ compared to the starting materials were used.

It has now surprisingly been found that also β-lactams are formed in substantially similar yields if these reactions are carried out in the presence of lower amounts of the suitable metal compound $MW(X)_a(Y)_b(Z)_c$.

Therefore, according to the invention a process is provided for the preparation of a trans-β-lactam compound of formula I,

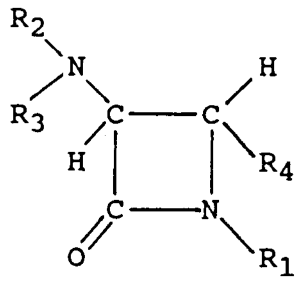

I

wherein

$R_2$ and $R_3$ are each hydrogen, (1-8C)alkyl, (1-18C)aryl or (1-8C)alkyl(1-18C)aryl, each optionally substituted with (1-8C)alkyl, (1-18C)aryl or (1-8C)alkyl(1-18C)aryl, or are

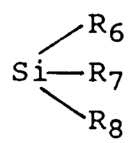

where $R_6$, $R_7$ and $R_8$ are each (1-8C)alkyl, (1-18C)aryl, (1-8C)alkyl(1-18C)aryl, each optionally substituted and $R_6$, $R_7$ and $R_8$ are the same or different, and $R_2$ and $R_3$ are the same or different, or $R_2$ and $R_3$ together with the nitrogen atom to which they are attached form a ring with up to 8 ring atoms, each optionally substituted with (1-8C)alkyl, (1-18C)aryl or (1-8C)alkyl(1-18C)aryl, $R_1$ is hydrogen, halogen, (1-8C)alkyl, (2-8C)alkenyl, (1-8C)alkoxy, (1-18C)aryl or (1-8C)alkyl(1-18C)aryl, or is

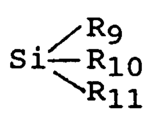

where $R_9$, $R_{10}$ and $R_{11}$ are each (1-8C)alkyl, (1-18C)aryl, (1-8C)alkyl(1-18C)aryl, each optionally substituted, and $R_9$, $R_{10}$ and $R_{11}$ are the same or different, or is

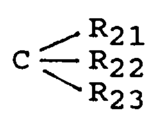

where $R_{21}$, $R_{22}$ and $R_{23}$ are each hydrogen, (1-8C)alkyl, (1-8C)alkoxy, (1-18C)aryl, (1-18C)aryloxy, (1-8C)-alkyl(1-18C)aryl, (1-18C)(1-8C)aralkoxy or heterocyclic, each optionally substituted, and $R_{21}$, $R_{22}$ and $R_{23}$ are the same or different,

$R_4$ is (1-8C)alkyl, hydroxy, halogen, sulphonyl, (1-8C)alkoxy, (2-8C)alkenyl, (2-8C)alkynyl or (1-18C)aryl, each optionally substituted, or is

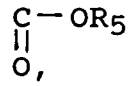

where $R_5$ is (1-8C)alkyl, or
$R_4$ is

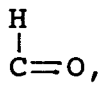 or 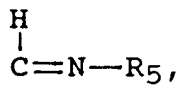

where $R_5$ is (1-8C)alkyl, (1-18C)aryl, (1-8C)alkyl(1-18C)aryl, or a heterocyclic group, each optionally substituted, or
$R_5$ is

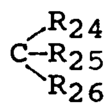

where $R_{24}$, $R_{25}$ and $R_{26}$ are each hydrogen, (1-8C)alkyl, (1-8C)alkoxy, (1-18C)aryl, (1-18C)aryloxy, (1-8C)-alkyl(1-18C)aryl, (1-18C)(1-8C)aralkoxy or heterocyclic, each optionally substituted, and $R_{24}$, $R_{25}$ and $R_{26}$ are the same or different, or
$R_4$ is a heterocyclic group, optionally substituted, by reacting a $R_2'R_3'$N-glycine ester of formula

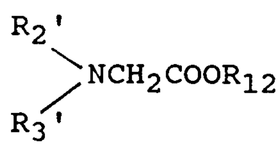

wherein
$R_{12}$ is (1-8C)alkyl, (1-8C)cycloalkyl(1-18C)aryl or (1-8C)alkyl(1-18C)aryl, each optionally substituted with (1-8C)alkyl, (1-8C)cycloalkyl(1-18C)aryl or (1-8C)alkyl(1-18C)aryl,
$R_2'$ and $R_3'$ is $R_2$ and $R_3$ respectively, with the proviso that when $R_2$ and $R_3$ are hydrogen, $R_2'$ and $R_3'$ form together with the nitrogen atom to which they are attached a ring of formula III

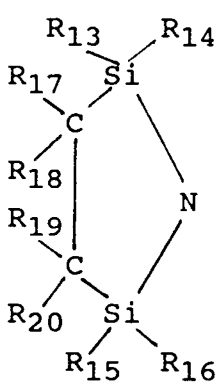

III

wherein
$R_{13}$, $R_{14}$, $R_{15}$, $R_{16}$, $R_{17}$, $R_{18}$, $R_{19}$ and $R_{20}$ are each (1-8C)alkyl, (1-18C)aryl, (1-8C)alkyl(1-18C)aryl, and $R_{13}$, $R_{14}$, $R_{15}$, $R_{16}$, $R_{17}$, $R_{18}$, $R_{19}$ and $R_{20}$ are the same or different,
with an imine of formula IV,

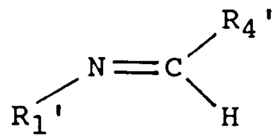

IV

where $R_4'$ is $R_4$, with the proviso that when $R_4$ is

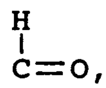 $R_4'$ is 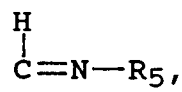

which group is hydrolysed by acid after the reaction,
and $R_1'$ is $R_1$ with the proviso that when $R_1$ is hydrogen $R_1'$ is

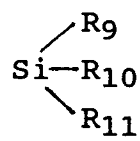

where $R_9$, $R_{10}$ and $R_{11}$ are defined as above, which group is hydrolysed by acid or base after the reaction,
where $R_1$, $R_4$ and $R_5$ are defined as for figure I,
and with the proviso that when $R_2$ and $R_3$ are both hydrogen, the ring of formula III is converted into $NH_2$ by acid or base catalysed hydrolysis,
in the presence of an alkali metal base and a metal compound of the formula $MW(X)_a(Y)_b(Z)_c$,
wherein
M is zinc, aluminum, zirconium, boron, tin or titanium,
X, Y and Z are (1-8C)alkyl, (1-18C)aryl, halide, alkoxide, thiolate, triflate or any other substituted sulfonate or any other suitable anionic group,
W is halide, thiolate, triflate or any other substituted sulfonate or any other suitable anionic group, and X, Y and Z are the same or different,
and a, b and c = 0-1, with the proviso that one of a, b or c is 1, all being integers,
characterized in that the reaction is carried out in the presence of an amount of $MW(X)_a(Y)_b(Z)_c$ which is lower than equimolar compared to the $R_2'R_3'$N-glycine ester.

The reactions are preferably carried out in inert, weakly polar solvents as for instance diethyl ether or tetrahydrofuran.

M in the metal compound $MW(X)_a(Y)_b(Z)_c$ is preferably zinc; as metal compound preferably $ZnCl_2$ is used. The values of a, b and c depend on the valency of M.

Advantageously, the compound $ZnW(X)_a(Y)_b(Z)_c$ is used in an amount of 0.1 to 0.5 equivalent compared to that of the $R_2'R_3'$N-glycine ester, preferably in an amount of 0.2 to 0.3 equivalent.

By the term "optionally substituted" unsubstituted or substituted with one or more groups "generally present in organic compounds" is meant, such as (1-8C)alkyl, particularly methyl, ethyl, propyl or butyl; (1 18C)aryl, particularly phenyl or naphthyl; (1-8C)alkyl(1-18C)aryl, particularly benzyl or xylyl; (1-8C)alkoxy, particularly methoxy, ethoxy, propoxy or butoxy; (1-18C)aryloxy, particularly phenoxy or naphthoxy; (1-18C)-(1-8C)aralkoxy, particularly benzyloxy or xylyloxy; heterocyclic, particularly pyridyl, furyl or thienyl; halogen, nitro, protected hydroxy or cyano.

With a heterocyclic group a 5 or 6 membered heterocyclic group is meant, particularly comprising a nitrogen, oxygen or sulfur atom.

A suitable anionic group comprises all anionic groups appropriate to form as ligand a complex with zinc, aluminum, zirconium, boron, tin or titanium, as indicated in formula II, or to form a salt with an alkali metal, such as halide, thiolate, triflate etc.

When an amount of metal compound lower than equimolar compared to the other starting materials is used, not only the yields are generally maintained on the same, nearly quantitative level, but the stereoselectivity, in favour of the trans compound, is even enhanced. Apparently, the metal compound

behaves as a catalyst resulting in a good yield and even a better stereoselectivity than when the reaction is carried out with a stoichiometric amount of metal compound as described in EP-A-0281177.
Another advantage is the use of less metal compound $MW(X)_a(Y)_b(Z)_c$ compared to the amount needed in the above-mentioned applications.

It is very important that the metal compounds (with the formula $MW(X)_a(Y)_b(Z)_c$) used in these reactions essentially are free of water. When small amounts of water are present, lower yields may be obtained and the stereoselectivity decreases.

The following non-limitative examples illustrate the invention.

General procedure

All reactions were carried out in an atmosphere of dry nitrogen using standard Schlenk-techniques ("Manipulations of air-sensitive compounds", D.F. Shriver, editor Mc. Graw Hill, New York, 1969). Solvents were distilled from sodium prior to use. 2,2,5,5-tetramethyl-1-aza-2,5-disilylacyclopentane-1-acetic acid ethyl ester was prepared according to the method described in Tetrahedron Lett., 22, 1787 (1981). Solutions of n-butyllithium in n-hexane and diethyl-zinc in n-hexane were obtained commercially.

It is very important to use a dry metal compound to prepare the metal enolates. Therefore for instance zinc dichloride was either prepared from zinc and dry hydrogen chloride in diethyl ether or commercially available zinc dichloride was dehydrated in refluxing thionyl chloride.

Example I

Preparation of 1-methyl-3-(2,2,5,5-tetramethyl-1-aza-2,5-disilacyclopentyl)-4-phenyl-2-azetidinone employing various amounts of zinc dichloride.

10 mmoles of n-butyllithium (6.67 ml of a 1.5 molar solution in hexane) was added to a stirred solution containing diisopropylamine (1.40 ml; 10 mmoles) and 25 ml of diethyl ether at -70 °C. This reaction mixture was stirred for 10 min and then 2,2,5,5-tetramethyl-1-aza-2,5-disilacyclopentane-1-acetic acid ethylester (2.45 g; 10 mmoles) was added. The solution was stirred for another 15 min at -70 °C and then 2.5 mmoles of zinc dichloride (2.5 ml of a 1.0 molar solution in diethyl ether) was added. After stirring for another 15 min at -70 °C 10 mmoles of N-(benzylidene)methylamine (1.19 g) was added. After 5 min at -70 °C the reaction mixture was slowly warmed to room temperature and stirred for another 8 hours, whereafter it was quenched with 20 ml of a saturated aqueous ammonium chloride solution. The water layer was extracted with diethyl ether. The diethyl ether extract was washed with water, dried with sodium sulfate and concentrated in vacuo to give 3.19 g (100%) of the pure 2-azetidinone product as a pale yellow solid. The $^1H$ NMR spectrum revealed that the product was a mixture of cis- and trans-isomers (cis/trans ratio 2:98). The results of experiments using other amounts of zinc dichloride are given in Table I.

## Table I

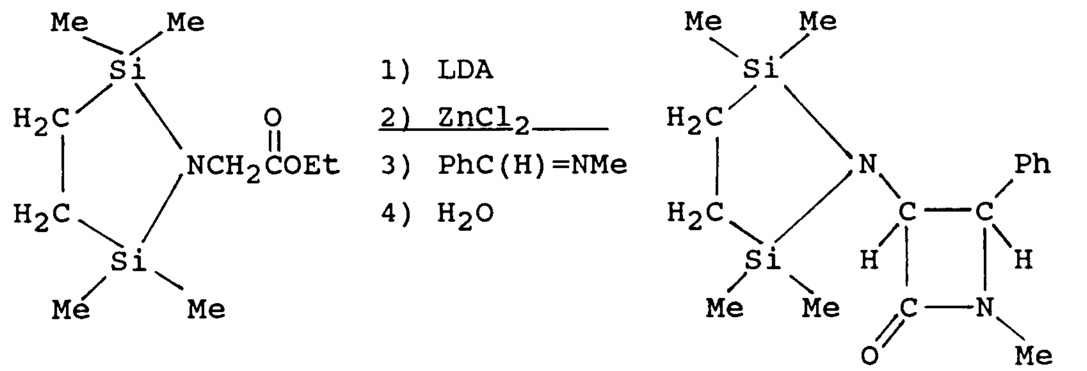


| amount of $ZnCl_2$ | | yield | cis | trans |
|---|---|---|---|---|
| 2 | equivalents | 65% | 10% | 90% |
| 1 | " | 98% | 8% | 92% |
| 0.5 | " | 95% | 4% | 96% |
| 0.25 | " | 100% | 2% | 98% |
| 0.1 | " | 80% | 2% | 98% |
| 0.00 | " | 0% | -- | -- |

Example II

Preparation of 1-benzyl-3-(2,2,5,5-tetramethyl-1-aza-2,5-disilacyclopentyl)-4-methyl-2-azetidinone employing a catalytic amount of zinc dichloride.

10 mmoles of n-butyllithium (6.67 ml of a 1.5 molar solution in hexane) was added to a stirred solution containing diisopropylamine (1.40 ml; 10 mmoles) and 25 ml of diethyl ether at -70 °C. This reaction mixture was stirred for 10 min and then 2,2,5,5-tetramethyl-1-aza-2,5-disilacyclopentane-1-acetic acid ethylester (2.45 g; 10 mmoles) was added. The solution was stirred for another 15 min at -70 °C and then 2.5 mmoles of zinc dichloride (2.5 ml of a 1.0 molar solution in diethyl ether) was added. After stirring for another 15 min at -70 °C 10 mmoles of N-(ethylidene)benzylamine (1.33 g) was added. After 5 min at -70 °C the reaction mixture was slowly warmed to room temperature and stirred for another 8 hours, whereafter it was quenched with 20 ml of a saturated aqueous ammonium chloride solution. The water layer was extracted with diethyl ether. The diethyl ether extract was washed with water, dried with sodium sulfate and concentrated in vacuo to give 3.00 g (90%) of the 2-azetidinone product as a pale yellow oil. The $^1H$ NMR spectrum revealed that the product was exclusively the trans-isomer.

Example III

Preparation of 1-methyl-3-(diethylamino)-4-phenyl-2-azetidinone employing a catalytic amount of zinc dichloride.

10 mmoles of n-butyllithium (6.67 ml of a 1.5 molar solution in hexane) was added to a stirred solution containing diisopropylamine (1.40 ml; 10 mmoles) and 30 ml of tetrahydrofuran at room temperature. This solution was stirred for 10 min and then N,N-diethylglycine ethylester (1.59 g; 10 mmoles) was added, which resulted in a pale yellow suspension. After stirring for another 15 min 2.5 mmoles of zinc dichloride (2.5 ml of a 1.0 molar solution in diethyl ether) were added, which resulted in a clear yellow solution. This solution was stirred for 30 min, whereafter some material (LiCl) precipitated. Then 10 mmoles of N-(benzylidene)methylamine (1.19 g) were added and the mixture was refluxed for 16 hours, whereafter it was quenched with 20 ml of a saturated aqueous ammonium chloride solution. The water layer was extracted with diethyl ether. The combined extracts were dried with sodium sulfate and concentrated in vacuo to give 2.09 g (90%) of the 2-azetidinone product as a yellow solid. The $^{1}H$ NMR spectrum revealed that the product was a mixture of cis- and trans-isomers (cis/trans ratio 27:73).

Employing one equivalent of zinc dichloride in tetrahydrofuran a cis/trans ratio of 58:42 was found; yield 90%.

Example IV

Preparation of trans-1-t-butyl-3-(2,2,5,5-tetramethyl-1-aza-2,5-disilacyclopentyl)-4-N-t-butylimino-2-azetidinone

10 mmoles of n-butyllithium (6.67 ml of a 1.5 molar solution in hexane) was added to a stirred solution containing diisopropylamine (1.40 ml; 10 mmoles) and 25 ml of diethyl ether at -70 °C. This reaction mixture was stirred for 10 min and then 2,2,5,5-tetramethyl-1-aza-2,5-disilacyclopentane-1-acetic acid ethylester (2.45 g; 10 mmoles) was added. The solution was stirred for another 15 min at -70 °C and then 2.5 mmoles of zinc dichloride (2.5 ml of a 1.0 molar solution in diethylether) was added. After 5 min at -70 °C, a white solid (LiCl) began to precipitate. Then bis-t-butyl-1,4-diaza-1,3-butadiene (1.68 g; 10 mmoles) was added. The reaction mixture was stirred for another 15 min at -70 °C and, after being warmed to room temperature, quenched with 20 ml of a saturated aqueous ammonium chloride solution. The water layer was extracted with diethyl ether. The diethyl ether extract was washed with water, dried with sodium sulfate and concentrated in vacuo to give 2.87 g (78%) of the pure 2-azetidinone product as a pale yellow solid.

Following the same procedure and using the same reaction conditions as used above, one more 3-(2,2,5,5-tetramethyl-1-aza-2,5-disilacyclopentyl) trans-$\beta$-lactam was obtained, see Table II.

The $^{1}H$ NMR and $^{13}C$ NMR spectra revealed that substantially only the respective trans-compounds were formed.

Table II

| | | yield | cis | trans |
|---|---|---|---|---|
| $R_1$ = t-butyl | $R_4$ = (t-butyl)imino | 78% | 0% | 100% |
| $R_1$ = t-butyl | $R_4$ = 2-pyridyl | 97% | 0% | 100% |

**Claims**

**1.** A process for the preparation of a trans-$\beta$-lactam compound of formula I,

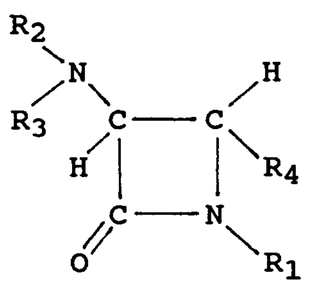

I

wherein

$R_2$ and $R_3$ are each hydrogen, (1-8C)alkyl, (1-18C)aryl or (1-8C)alkyl(1-18C)aryl, each optionally substituted with (1-8C)alkyl, (1-18C)aryl or (1-8C)alkyl(1-18C)aryl, or are

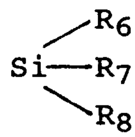

where $R_6$, $R_7$ and $R_5$ are each (1-8C)alkyl, (1-18C)aryl, (1-8C)alkyl(1-18C)aryl, each optionally substituted and $R_6$, $R_7$ and $R_8$ are the same or different,
and $R_2$ and $R_3$ are the same or different,
or $R_2$ and $R_3$ together with the nitrogen atom to which they are attached form a ring with up to 8 ring atoms, each optionally substituted with (1-8C)alkyl, (1-18C)aryl or (1-8C)alkyl(1-18C)aryl,
$R_1$ is hydrogen, halogen, (1-8C)alkyl, (2-8C)alkenyl, (1-8C)alkoxy, (1-18C)aryl or (1-8C)alkyl(1-18C)aryl, or is

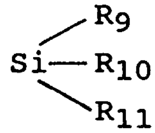

where $R_9$, $R_{10}$ and $R_{11}$ are each (1-8C)alkyl, (1-18C)aryl, (1-8C)alkyl(1-18C)aryl, each optionally substituted, and $R_9$, $R_{10}$ and $R_{11}$ are the same or different,
or is

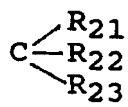

where $R_{21}$, $R_{22}$ and $R_{23}$ are each hydrogen, (1-8C)alkyl, (1-8C)alkoxy, (1-18C)aryl, (1-18C)aryloxy, (1-8C)alkyl(1-18C)aryl, (1-18C)(1-8C)aralkoxy or heterocyclic, each optionally substituted, and $R_{21}$, $R_{22}$ and $R_{23}$ are the same or different,
$R_4$ is (1-8C)alkyl, hydroxy, halogen, sulphonyl, (1-8C)alkoxy, (2-8C)alkenyl, (2-8C)alkynyl or (1-18C)aryl, each optionally substituted, or is

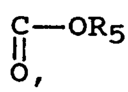

where $R_5$ is (1-8C)alkyl, or
$R_4$ is

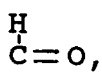 or 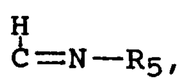

where $R_5$ is (1-8C)alkyl, (1-18C)aryl, (1-8C)alkyl(1-18C)aryl, or a heterocyclic group, each optionally substituted, or
$R_5$ is

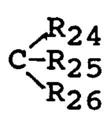

where $R_{24}$, $R_{25}$ and $R_{26}$ are each hydrogen, (1-8C)alkyl, (1-8C)alkoxy, (1-18C)aryl, (1-18C)aryloxy, (1-8C)alkyl(1-18C)aryl, (1-18C)(1-8C)aralkoxy or heterocyclic, each optionally substituted, and $R_{24}$, $R_{25}$ and $R_{26}$ are the same or different, or

$R_4$ is a heterocyclic group, optionally substituted, by reacting a $R_2'R_3'$N-glycine ester of formula

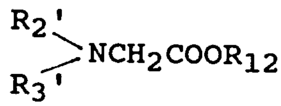

wherein
$R_{12}$ is (1-8C)alkyl, (1-8C)cycloalkyl(1-18C)aryl or (1-8C)alkyl(1-18C)aryl, each optionally substituted with (1-8C)alkyl, (1-8C)cycloalkyl(1-18C)aryl or (1-8C)alkyl(1-18C)aryl,
$R_2'$ and $R_3'$ is $R_2$ and $R_3$ respectively, with the proviso that when $R_2$ and $R_3$ are hydrogen, $R_2'$ and $R_3'$ form together with the nitrogen atom to which they are attached a ring of formula III

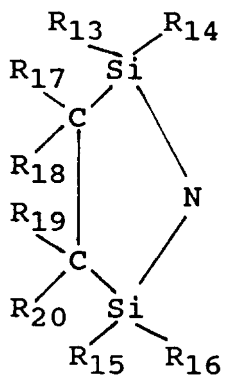

III

wherein
$R_{13}$, $R_{14}$, $R_{15}$, $R_{16}$, $R_{17}$, $R_{18}$, $R_{19}$ and $R_{20}$ are each (1-8C)alkyl, (1-18C)aryl, (1-8C)alkyl(1-18C)aryl, and $R_{13}$, $R_{14}$, $R_{15}$, $R_{16}$, $R_{17}$, $R_{18}$, $R_{19}$ and $R_{20}$ are the same or different,
with an imine of formula IV,

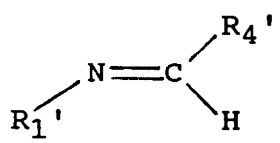

IV

where $R_4'$ is $R_4$, with the proviso that when $R_4$ is

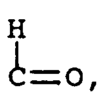, $R_4'$ is 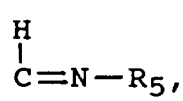,

which group is hydrolysed by acid after the reaction,
and $R_1'$ is $R_1$ with the proviso that when $R_1$ is hydrogen $R_1'$ is

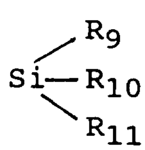

where $R_9$, $R_{10}$ and $R_{11}$ are defined as above, which group is hydrolysed by acid or base after the reaction,

where $R_1$, $R_4$ and $R_5$ are defined as for figure I,
and with the proviso that when $R_2$ and $R_3$ are both hydrogen, the ring of formula III is converted into $NH_2$ by acid or base catalysed hydrolysis,
in the presence of an alkali metal base and a metal compound of the formula $MW(X)_a(Y)_b(Z)_c$,
wherein
M is zinc, aluminum, zirconium, boron, tin or titanium,
X, Y and Z are (1-8C)alkyl, (1-18C)aryl, halide, alkoxide, thiolate, triflate or any other substituted sulfonate or any other suitable anionic group,
W is halide, thiolate, triflate or any other substituted sulfonate or any other suitable anionic group, and X, Y and Z are the same or different,
and a, b and c = 0-1, with the proviso that one of a, b or c is 1, all being integers,
characterized in that the reaction is carried out in the presence of an amount of $MW(X)_a(Y)_b(Z)_c$ which is lower than equimolar compared to the $R_2'R_3'$N-glycine ester.

**2.** A process according to claim 1, characterized by the preparation of a trans-$\beta$-lactam of formula I, as depicted in claim 1,
wherein
$R_2$ and $R_3$ together with the nitrogen atom to which they are attached form 2,2,5,5-tetramethyl-1-aza-2,5-disilacyclopentyl or
$R_2$ and $R_3$ are hydrogen, and
$R_1$ is methyl and $R_4$ is phenyl or
$R_1$ is benzyl and $R_4$ is methyl or
$R_1$ is butyl and $R_4$ is N-t-butylimino or
$R_1$ is butyl and $R_4$ is 2-pyridyl,
$R_2$ is ethyl, $R_3$ is ethyl and
$R_1$ is methyl, $R_4$ is phenyl.

**3.** A process according to claim 1 or 2, characterized in that the reaction is carried out in an inert, weakly polar solvent.

**4.** A process according to claim 3, characterized in that the reaction is carried out in diethyl ether or tetrahydrofuran.

**5.** A process according to any one of the claims 1-4, characterized in that in the formula $MW(X)_a(Y)_b(Z)_c$ M is zinc.

**6.** A process according to claim 5, characterized in that $ZnW(X)_a(Y)_b(Z)_c$ is $ZnCl_2$.

**7.** A process according to claim 5 or 6, characterized in that $ZnW(X)_a(Y)_b(Z)_c$ is used in an amount of 0.1 to 0.5 equivalent compared to the amount of the $R_2'R_3'$N-glycine ester, where W, X, Y, Z, a, b, c, $R_2'$ and $R_3'$ are as defined in claim 1.

**8.** A process according to claim 7, characterized in that $ZnW(X)_a(Y)_b(Z)_c$ is used in an amount of 0.2 to 0.3 equivalent compared to the amount of the $R_2'R_3'$N-glycine ester.

**Patentansprüche**

**1.** Verfahren zur Herstellung einer trans-beta-Lactamverbindung der Formel I

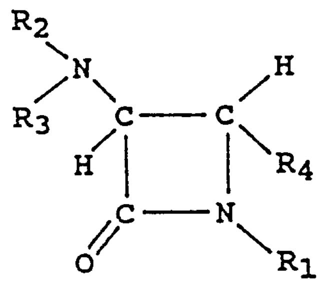

I

worin
$R_2$ und $R_3$ jeweils für Wasserstoff, (1-8C)-Alkyl, (1-18C)-Aryl oder (1-8C)-Alkyl-(1-18C)-aryl, die jeweils gegebenenfalls durch (1-8C)-Alkyl, (1-18C)-Aryl oder (1-8C)-Alkyl-(1-18C)-aryl substituiert sind, oder für

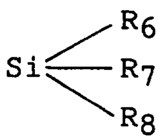

stehen, wobei $R_6$, $R_7$ und $R_8$ jeweils für (1-8C)-Alkyl, (1-18C)-Aryl, (1-8C)-Alkyl-(1-18C)-Aryl stehen, die jeweils gegebenenfalls substituiert sind, und $R_6$, $R_7$ und $R_8$ gleich oder verschieden sind, und $R_2$ und $R_3$ gleich oder verschieden sind oder $R_2$ und $R_3$ zusammen mit dem Stickstoffatom, an das sie gebunden sind, einen Ring mit bis zu 8 Ringatomen bilden, die jeweils gegebenenfalls durch (1-8C)-Alkyl, (1-18C)-Aryl oder (1-8C)Alkyl-(1-18C)-aryl substituiert sind,
$R_1$ für Wasserstoff, Halogen, (1-8C)-Alkyl, (2-8C)-Alkenyl, (1-8C)-Alkoxy, (1-18C)-Aryl oder (1-8C)-Alkyl-(1-18C)-aryl steht oder für

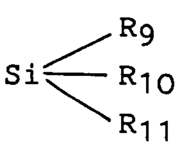

steht, wobei $R_9$, $R_{10}$ und $R_{11}$ jeweils für (1-8C)-Alkyl, (1-18C)-Aryl, (1-8C)-Alkyl-(1-18C)-aryl stehen, die jeweils gegebenenfalls substituiert sind, und $R_9$, $R_{10}$ und $R_{11}$ gleich oder verschieden sind, oder für

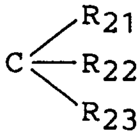

steht, wobei $R_{21}$, $R_{22}$ und $R_{23}$ jeweils für Wasserstoff, (1-8C)-Alkyl, (1-8C)-Alkoxy, (1-18C)-Aryl, (1-18C)-Aryloxy, (1-8C)-Alkyl-(1-18C)-aryl, (1-18C)-(1-8C)-Aralkoxy oder eine heterocyclische Gruppe stehen, die jeweils gegebenenfalls substituiert sind, und $R_{21}$, $R_{22}$ und $R_{23}$ gleich oder verschieden sind,
$R_4$ für (1-8C)-Alky1, Hydroxyl, Halogen, Sulfonyl, (1-8C)-Alkoxy, (2-8C)-Alkenyl, (2-8C)-Alkinyl oder (1-18)-Aryl steht, die jeweils gegebenenfalls substituiert sind, oder für

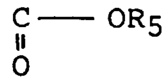

steht, wobei $R_5$ für (1-8C)-Alkyl steht, oder $R_4$ für

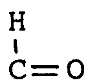 oder 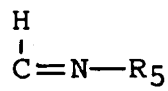

steht,
wobei $R_5$ für (1-8C)-Alkyl, (1-18C)-Aryl, (1-8C)-Alkyl-(1-18C)-aryl oder eine heterocyclische Gruppe steht, die jeweils gegebenenfalls substituiert sind, oder $R_5$ für

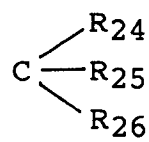

steht, wobei $R_{24}$, $R_{25}$ und $R_{26}$ jeweils für Wasserstoff, (1-8C)-Alkyl, (1-8C)-Alkoxy, (1-18C)-Aryl, (1-18C)-Aryloxy, (1-8C)-Alkyl-(1-18C)-aryl, (1-18C)-(1-8C)-Aralkoxy oder eine heterocyclische Gruppe stehen, die jeweils gegebenenfalls substituiert sind, und $R_{24}$, $R_{25}$ und $R_{26}$ gleich oder verschieden sind, oder
$R_4$ für eine gegebenenfalls substituierte heterocyclische Gruppe steht,
durch Umsetzung eines $R_2'R_3'$N-Glycinesters der Formel

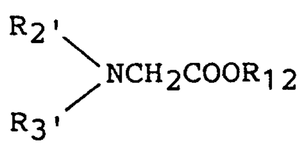

worin
$R_{12}$ für (1-8C)-Alkyl, (1-8C)-Cycloalkyl-(1-18C)-aryl oder (1-8C)-Alkyl-(1-18C)-aryl steht, die jeweils gegebenenfalls durch (1-8C)-Alkyl, (1-8C)-Cycloalkyl-(1-18C)-aryl oder (1-8C)-Alkyl-(1-18C)-aryl substituiert sind,
$R_2'$ und $R_3'$ für $R_2$ bzw. $R_3$ stehen, unter der Bedingung, dass, wenn $R_2$ und $R_3$ für Wasserstoff stehen, $R_2'$ und $R_3'$ zusammen mit dem Stickstoffatom, an das sie gebunden sind, einen Ring der Formel III

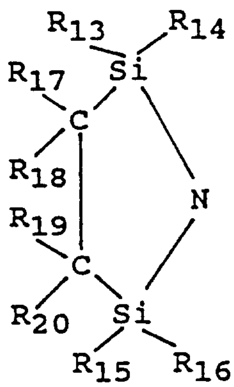

III

bilden, worin $R_{13}$, $R_{14}$, $R_{15}$, $R_{16}$, $R_{17}$, $R_{18}$, $R_{19}$ und $R_{20}$ jeweils für (1-8C)-Alkyl, (1-18C)-Aryl, (1-8C)-Alkyl-(1-18C)-aryl stehen und $R_{13}$, $R_{14}$, $R_{15}$, $R_{16}$, $R_{17}$, $R_{18}$, $R_{19}$ und $R_{20}$ gleich oder verschieden sind, mit einem Imin der Formel IV

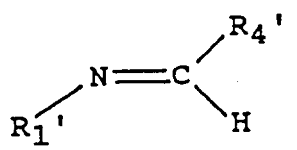

IV

worin
$R_4'$ für $R_4$ steht, unter der Bedingung, dass wenn $R_4$ für

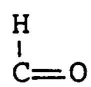

steht, $R_4'$ für

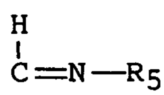

steht, welche Gruppe nach der Reaktion durch Säure hydrolysiert wird, und
$R_1'$ für $R_1$ steht, unter der Bedingung, dass, wenn $R_1$ für Wasserstoff steht, $R_1'$ für

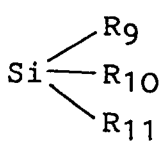

steht, wobei $R_9$, $R_{10}$ und $R_{11}$ wie oben definiert sind, welche Gruppe nach der Reaktion durch Säure oder Base hydrolysiert wird,
wobei $R_1$, $R_4$ und $R_5$ wie für Formel I definiert sind, und unter der Bedingung, dass, wenn $R_2$ und $R_3$ beide für Wasserstoff stehen, der Ring der Formel III durch säure- oder basenkatalysierte Hydrolyse in $NH_2$ übergeführt wird,

in Gegenwart einer Alkalimetallbase und einer Metallverbindung der Formel

$MW(X)_a(Y)_b(Z)_c$

worin
M für Zink, Aluminium, Zirkonium, Bor, Zinn oder Titan steht,
X, Y und Z für (1-8C)-Alkyl, (1-18C)Aryl, Halogenid, Alkanolat, Thiolat, Triflat oder ein beliebiges anderes substituiertes Sulfonat oder eine beliebige andere geeignete anionische Gruppe stehen,
W für Halogenid, Thiolat, Triflat oder ein beliebiges anderes substituiertes Sulfonat oder eine beliebige andere geeignete anionische Gruppe steht und
X, Y und Z gleich oder verschieden sind und
a, b und c = 0 bis 1, unter der Bedingung, dass eines von a, b oder c für 1 steht, wobei alle ganze Zahlen sind,
dadurch gekennzeichnet, dass die Reaktion in Gegenwart einer Menge an $MW(X)_a(Y)_b(Z)_c$ ausgeführt wird, die geringer als äquimolar, verglichen mit dem $R_2$'$R_3$'N-Glycinester, ist.

**2.** Verfahren nach Anspruch 1, gekennzeichnet durch die Herstellung eines trans-beta-Lactams der Formel I, wie sie in Anspruch 1 wiedergegeben ist, worin
$R_2$ und $R_3$ zusammen mit dem Stickstoffatom, an das sie gebunden sind, 2,2,5,5-Tetramethyl-1-aza-2,5-disilacylopentyl bilden oder
$R_2$ und $R_3$ für Wasserstoff stehen und
$R_1$ für Methyl steht und $R_4$ für Phenyl steht oder
$R_1$ für Benzyl steht und $R_4$ für Methyl steht oder
$R_1$ für Butyl steht und $R_4$ für N-t-Butylimino steht oder
$R_1$ für Butyl steht und $R_4$ für 2-Pyridyl steht,
$R_2$ für Ethyl steht, $R_3$ für Ethyl steht und
$R_1$ für Methyl steht, $R_4$ für Phenyl steht.

**3.** Verfahren nach Anspruch 1 oder 2, dadurch gekennzeichnet, dass die Reaktion in einem inerten, schwach polaren Lösungsmittel ausgeführt wird.

**4.** Verfahren nach Anspruch 3, dadurch gekennzeichnet, dass die Reaktion in Diethylether oder Tetrahydrofuran ausgeführt wird.

**5.** Verfahren nach einem der Ansprüche 1 bis 4, dadurch gekennzeichnet, dass M in der Formel $MW(X)_a$-$(Y)_b(Z)_c$ für Zink steht.

**6.** Verfahren nach Anspruch 5, dadurch gekennzeichnet, dass $ZnW(X)_a(Y)_b(Z)_c$ für $ZnCl_2$ steht.

**7.** Verfahren nach Anspruch 5 oder 6, dadurch gekennzeichnet, dass $ZnW(X)_a(Y)_b(Z)_c$ in einer Menge von 0,1 bis 0,5 Aequivalent, verglichen mit der Menge des $R_2$'$R_3$'N-Glycinesters, verwendet wird, wobei W, X, Y, Z, a, b, c, $R_2$' und $R_3$' wie in Anspruch 1 definiert sind.

**8.** Verfahren nach Anspruch 7, dadurch gekennzeichnet, dass $ZnW(X)_a(Y)_b(Z)_c$ in einer Menge von 0,2 bis 0,3 Aequivalent, verglichen mit der Menge des $R_2$'$R_3$'N-Glycinesters, verwendet wird.

**Revendications**

**1.** Procédé de préparation d'un trans-$\beta$-lactame de formule I :

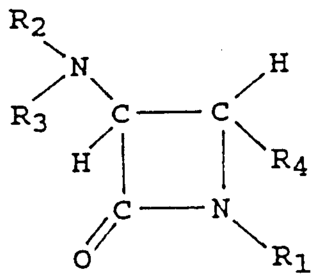

(I)

où

$R_2$ et $R_3$ sont chacun hydrogène, (1-8C)alcoyle, (1-18C)aryle ou (1-8)alcoyl(1-18C)aryle, chacun facultativement substitué par (1-8C)alcoyle, (1-18C)aryle ou (1-8C)alcoyl(1-18C)aryle, ou sont

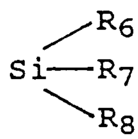

où $R_6$, $R_7$ et $R_8$ sont chacun (1-8C)alcoyle, (1-18C)aryle, (1-8C)alcoyl(1-18C)aryle, chacun facultativement substitué et $R_6$, $R_7$ et $R_8$ sont identiques ou différents,

et $R_2$ et $R_3$ sont identiques ou différents,

ou $R_2$ et $R_3$ conjointement avec l'atome d'azote auquel ils sont unis forment un cycle comptant jusqu'à 8 atomes de cycle, chacun facultativement substitué par (1-8C)alcoyle, (1-18C)aryle ou (1-8C)-alcoyl(1-18C)-aryle,

$R_1$ est hydrogène, halogène, (1-8C)alcoyle, (2-8C)alcényle, (1-8C)alcoxy, (1-18C)aryle ou (1-8C)-alcoyl(1-18C)aryle, ou est

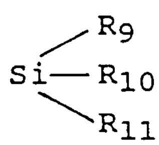

où $R_9$, $R_{10}$ et $R_{11}$ sont chacun (1-8C)alcoyle, (1-18C)aryle, (1-8C)alcoyl(1-18C)aryle, chacun facultativement substitué et $R_9$, $R_{10}$ et $R_{11}$ sont identiques ou différents, ou est

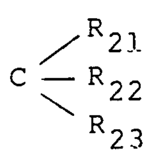

où $R_{21}$, $R_{22}$ et $R_{23}$ sont chacun hydrogène, (1-8C)alcoyle, (1-8C)alcoxy, (1-18C)aryle, (1-18C)aryloxy,

(1-8C)alcoyl(1-18C)aryle, (1-18C)(1-8C)aralcoxy ou hétérocyclique, chacun facultativement substitué et $R_{21}$, $R_{22}$ et $R_{23}$ sont identiques ou différents,

$R_4$ est (1-8C)alcoyle, hydroxyle, halogène, sulfonyle, (1-8C)alcoxy, (2-8C)alcényle, (2-8C)alcynyle ou (1-18C)aryle, chacun facultativement substitué, ou est $O=C\text{-}OR_5$, où $R_5$ est un (1-8C)alcoyle, ou

$R_4$ est $H\text{-}C=O$ ou $H\text{-}C=N\text{-}R_5$,

où $R_5$ est (1-8C)alcoyle, (1-18C)aryle, (1-8C)alcoyl(1-18C)aryle ou un radical hétérocyclique, chacun facultativement substitué, ou $R_5$ est

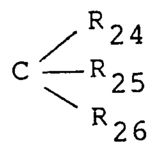

où $R_{24}$, $R_{25}$ et $R_{26}$ sont chacun hydrogène, (1-8C)alcoyle, (1-8C)alcoxy, (1-18C)aryle, (1-18C)aryloxy, (1-8C)alcoyl(1-18C)aryle, (1-18C)(1-8C)aralcoxy ou hétérocyclique, chacun facultativement substitué, et $R_{24}$, $R_{25}$ et $R_{26}$ sont identiques ou différents, ou

$R_4$ est un radical hétérocyclique facultativement substitué,

par réaction d'un ester de $R_2'R_3'$N-glycine de formule :

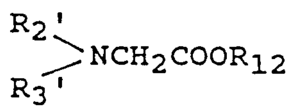

où

$R_{12}$ est (1-8C)alcoyle, (1-8C)cycloalcoyl(1-18C)aryle ou (1-8C)alcoyl(1-18C)aryle, chacun facultativement substitué par (1-8C)alcoyle, (1-8C)cycloalcoyl(1-18C)aryle ou (1-8C)alcoyl(1-18C)aryle,

$R_2'$ et $R_3'$ sont $R_2$ et $R_3$ respectivement, avec la restriction que lorsque $R_2$ et $R_3$ sont hydrogène, $R_2'$ et $R_3'$ forment ensemble avec l'atome d'azote auquel ils sont unis un cycle de formule III :

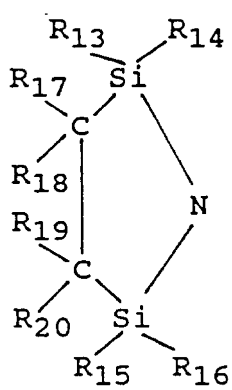

(III)

où $R_{13}$, $R_{14}$, $R_{15}$, $R_{16}$, $R_{17}$, $R_{18}$, $R_{19}$ et $R_{20}$ sont chacun (1-8C)alcoyle, (1-18C)aryle, (1-8C)alcoyl(1-18C)aryle et $R_{13}$, $R_{14}$, $R_{15}$, $R_{16}$, $R_{17}$, $R_{18}$, $R_{19}$ et $R_{20}$ sont identiques ou différents,

avec une imine de formule IV :

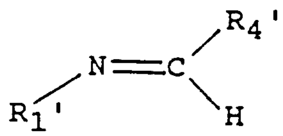

(IV)

où

$R_4$' est $R_4$, avec la restriction que lorsque $R_4$ est H-C=O, $R_4$' est H-C=N-$R_5$, lequel radical est hydrolysé par un acide après la réaction,

et $R_1$' est $R_1$ avec la restriction que lorsque $R_1$ est hydrogène, $R_1$' est

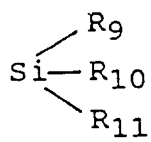

où $R_9$, $R_{10}$ et $R_{11}$ sont tels que définis ci-dessus,

lequel radical est hydrolysé par un acide ou une base après la réaction,

où

$R_1$, $R_4$ et $R_5$ sont tels que définis à propos de la formule I et avec la restriction que lorsque $R_2$ et $R_3$ sont tous deux hydrogène, le cycle de la formule III est converti en $NH_2$ par hydrogénolyse catalysée par un acide ou une base

en présence d'une base de métal alcalin et d'un composé métallique de formule :

$MW(X)_a(Y)_b(Z)_c$

où

M est le zinc, l'aluminium, le zirconium, le bore, l'étain ou le titane,

X, Y et Z sont (1-8C)alcoyle, (1-18C)aryle, halogénure, alcoolate, thiolate, triflate ou tout autre sulfonate substitué ou tout autre radical anionique approprié,

W est halogénure, thiolate, triflate ou tout autre sulfonate substitué ou tout autre radical anionique approprié et X, Y et Z sont identiques ou différents,

et a, b, c = 0 ou 1, avec la restriction que l'un d'entre a, b et c est 1, tous étant des entiers,

caractérisé en ce que la réaction est exécutée en présence d'une quantité de $MW(X)_a(Y)_b(Z)_c$ qui est moins que équimoléculaire par comparaison avec l'ester de $R_2$'$R_3$'N-glycine.

2. Procédé suivant la revendication 1, caractérisé par la préparation d'un trans-$\beta$-lactame de formule I, suivant la revendication 1,
   où
   $R_2$ et $R_3$ conjointement avec l'atome d'azote auquel ils sont unis forment un radical 2,2,5,5-tétraméthyl-1-aza-2,5-disilacyclopentyle, ou
   $R_2$ et $R_3$ sont hydrogène, et
   $R_1$ est méthyle et $R_4$ est phényle, ou
   $R_1$ est benzyle et $R_4$ est méthyle, ou
   $R_1$ est butyle et $R_4$ est N-t-butylimino, ou
   $R_1$ est butyle et $R_4$ est 2-pyridyle,
   $R_2$ est éthyle, $R_3$ est éthyle, et
   $R_1$ est méthyle, $R_4$ est phényle.

3. Procédé suivant la revendication 1 ou 2, caractérisé en ce que la réaction est exécutée dans un solvant inerte faiblement polaire.

4. Procédé suivant la revendication 3, caractérisé en ce que la réaction est exécutée dans l'éther diéthylique ou le tétrahydrofuranne.

5. Procédé suivant l'une quelconque des revendications 1 à 4, caractérisé en ce que M est le zinc dans la formule $MW(X)_a(Y)_b(Z)_c$.

6. Procédé suivant la revendication 5, caractérisé en ce que $ZnW(X)_a(Y)_b(Z)_c$ est $ZnCl_2$.

7. Procédé suivant la revendication 5 ou 6, caractérisé en ce que $ZnW(X)_a(Y)_b(Z)_c$ est utilisé en une quantité de 0,1 à 0,5 équivalent par rapport à la quantité de l'ester de $R_2'R_3'$N-glycine, où W, X, Y, Z, a, b, c, $R_2'$ et $R_3'$ sont tels que définis dans la revendication 1.

8. Procédé suivant la revendication 7, caractérisé en ce que $ZnW(X)_a(Y)_b(Z)_c$ est utilisé en une quantité de 0,2 à 0,3 équivalent par rapport à la quantité d'ester de $R_2'R_3'$N-glycine.